# EUROPEAN PATENT APPLICATION

(11) **EP 4 470 604 A1**
(43) Date of publication of application: **04.12.2024**
(21) Application number: 23176287.3
(22) Date of filing: 31.05.2023
(51) Int. Cl.: A61N 1/375

(54) **REFERENCE MARKERS ON A HEADER**

(71) Applicant: BIOTRONIK SE & Co. KG, 12359 Berlin (DE)
(72) Inventor: Litzke, Jan, 13507 Berlin (DE); Zastrow, Aljoscha, 12103 Berlin (DE)
(74) Representative: Biotronik Corporate Services SE

(57) **Abstract**

The present invention relates to an implantable medical device (1), comprising a housing (2) enclosing an electronic circuit of the implantable medical device (1), the housing (2) comprising a feedthrough (3) having at least one feedthrough conductor (30) for electrically connecting the electronic circuit from outside the housing (2), and a header (4), the header (4) comprising a header core (40) having at least one receptacle (41) for receiving a connector of an electrode lead and at least one electrical conductor (42) for connecting an electrical contact (43) of the header core (40) to said at least one feedthrough conductor (30) of the feedthrough (3). According to the present invention, the implantable medical device (1) comprises an optical and/or mechanical reference marker (10) arranged on the header (4) or the housing (2), wherein the reference marker (10) is configured to allow automated alignment of the header core (40) with respect to the housing (2) during production of the implantable medical device (1) so that said at least one electrical conductor (42) contacts the at least one feedthrough conductor (30) of the feedthrough (3).

## Description

For automated production of permanent medical implants such as implantable pacemakers, implantable cardioverter defibrillators, implantable neurostimulators and the like, usually the difficulty arises to precisely automatically position a header of the respective implant, via which header electrodes are e.g. connectable to the medical implant, with respect to a housing of the implant in order to electrically connect the header to electrical contacts (e.g. pins) of a feedthrough of said housing of the medical implant.

Often, orientation without a reference plane is difficult because the reference edges are either hidden by the header or complicated by the tolerance chains and do not guarantee sufficient process reliability.

In the case of automation, it is necessary to achieve the highest possible repeatability of the assembly processes. Particularly in case a potting process is used when fabricating a header as well as a subsequent polishing process, each header is individually designed which complicates alignment of header and housing of the medical implant. Furthermore, mounting components to the header is challenging in case the header comprises variations in size and shape due to being e.g., casted with a potting compound around a header core.

Based on the above, the problem to be solved by the present application is to provide a medical implant, a medical implant assembly, and a method for producing a medical implant that allow to improve automatic alignment of header or header core and housing during production of the medical implant. Particularly, further objectives of the present invention can relate to improvement of process results, reduction of scrap, shortening process development, and reduction of fixture development time and cost.

This problem is solved by a medical implant having the features of claim 1, a medical implant assembly having the features of claim 13, and a method having the features of claim 14.

According to claim 1, a medical implant is disclosed, comprising:
- a housing enclosing an electronic circuit of the medical device, the housing comprising a feedthrough having at least one feedthrough conductor, particularly a feedthrough pin, for electrically connecting the electronic circuit from outside the housing, and
- a header, the header comprising a header core having at least one receptacle for receiving a connector of an electrode lead and at least one electrical conductor for connecting an electrical contact of the header core to said at least one feedthrough conductor of the feedthrough.

Particularly, said electrical contact may electrically be connected to the receptacle/electrode lead and thus provides a connection between the electrode lead and the electrical circuit inside said housing.

According to the invention, the implantable medical device comprises an optical and/or mechanical reference marker arranged on the header or on a housing, wherein the reference marker is configured to allow automated alignment of the header or header core with respect to the housing during production of the implantable medical device so that said at least one electrical conductor contacts the at least one feedthrough conductor of the feedthrough.

Thus, due to the optical reference marker, the correct assembly position of the header or header core may be found by an automatic assembly machine, which is configured to permanently assign the reference marker to a reference edge on the housing. Otherwise in case the reference marker is arranged on the housing, it may be permanently assigned to a reference edge of the header core or header. Such reference edge on the housing may be formed by a manufacturer's name or brand or a product name, label or code (e.g., alphanumeric and/or barcode) arranged on the housing, e.g. introduced my means of laser inscription on the housing material.

Advantageously, with a reference marker, a higher process accuracy is achieved, which contributes to a reduction of scrap and rework during production. Particularly, the reference marker allows to exactly position the header core with respect to the housing along at least two linear independent axes / directions such as the X-axis and the Y-axis. Particularly, the aim of the present invention is to provide an assembly machine with an orientation in the X, Y and, if possible, Z axis in order to achieve the highest possible positional accuracy and thus a high degree of assembly process reliability.

According to a preferred embodiment, the reference marker is arranged on the header, particularly on the header core. Particularly, the header core may be surrounded by a potting or molding compound which provides an outer surface of the header. The potting or molding compound may be transparent, e.g., such an epoxy resin or a thermoplastic polyurethane. The reference marker may be arranged on the header core and may be visible through the potting or molding compound. However, the reference marker may also be arranged on the potting compound, i.e. form a portion of an outer surface of the header. Particularly, the potting compound may be applied onto the header core to form the header once the header core is automatically positioned with respect to the housing/feedthrough based on the reference marker. For this, header core and housing may be arranged in a mold.

Furthermore, according to a preferred embodiment, the reference marker may be arranged on said electrical conductor, which may be a wiring band. Furthermore, according to a preferred embodiment, the reference marker may be arranged on a printed circuit board of the header.

Furthermore, in an embodiment, arranging the reference marker on the header, particularly header core, or an any other suitably place may be done by one of: potting (e.g. with an epoxy resin), injection molding, gluing, plugging, welding (e.g. ultrasonic welding), machining, spraying, printing.

According to a preferred embodiment, it is also possible to provide the reference marker on the housing (e.g., similar to the X-ray detection). This option is useful for headers that do not have header cores, inserts or other mounting aids in the header. In other words, a pure potting or molding header.

Furthermore, according to a preferred embodiment, the reference marker is arranged on an anchor of the housing. Particularly, the anchor anchors the header to the housing, particularly by means of a potting or molding material of the header, wherein the anchor may be embedded in the potting or molding material to secure the header to the housing.

According to another preferred embodiment, the reference marker is arranged on the feedthrough of the housing.

Furthermore, according to a further preferred embodiment, the reference marker is arranged on a flange of the housing, wherein the feedthrough is fixed to the flange.

According to yet another preferred embodiment, the reference marker is arranged directly on the reference edge of the housing.

According to a further preferred embodiment, the reference marker is formed (e.g., by injection molding) directly on the header, particularly via a master or injection molding tool, such that the reference marker comprises a 3D surface and therefore forms a mechanical and/or optical reference marker, in particular.

According to a preferred embodiment, the reference marker comprises at least one of: a 3D shape, one or several graphical features, one or several lines, one or several points, one or several circles, one or several colors, an image (e.g. of a registered trademark), a feature detectable by X-ray. These afore-mentioned features may be combined with one another in any sensible way. Particularly, these features may be combined to provide a mechanically, i.e. in form of a mechanical contact point, as well as optically recognizable reference marker.

According to a preferred embodiment, the reference marker is a high contrast reference marker and preferably comprises at least one black portion. According to a preferred embodiment, the reference marker defines a circle, the circle comprising two relatively dark sectors (e.g. black sectors) and two relatively bright sectors being brighter than the dark sectors, wherein the relatively dark and relatively bright sectors are arranged in an alternating fashion. Preferably each sector corresponds to an angle of 90°, i.e., corresponds to ¼ of the circle's area.

According to a further aspect of the present invention, an implantable medical device assembly is disclosed, comprising:
- a housing enclosing an electronic circuit of the medical device, the housing comprising a feedthrough having at least one feedthrough conductor, particularly a feedthrough pin, for electrically connecting the electronic circuit from outside the housing, and
- a header, the header comprising a header core having at least one receptacle for receiving a connector of an electrode lead and at least one electrical conductor for connecting an electrical contact of the header core to said at least one feedthrough conductor of the feedthrough (particularly so as to connect the receptacle to the at least one feedthrough conductor).

According to the invention, the implantable medical device assembly comprises a mandrel configured to be inserted into said receptacle to prevent ingress of potting or molding compound into the receptacle upon casting the header with a potting or molding compound, and wherein an optical and/or mechanical reference marker is arranged on the mandrel, wherein the reference marker is configured to allow automated alignment of the header core with respect to the housing so that said at least one electrical conductor contacts the at least one feedthrough conductor of the feedthrough.

According to yet another aspect of the present invention, a method for producing an implantable medical device is disclosed, the method comprising the steps of:
- providing a housing of the implantable medical device enclosing an electronic circuit of the implantable medical device, the housing comprising a feedthrough having at least one feedthrough conductor, particularly a feedthrough pin, for electrically connecting the electronic circuit from outside the housing,
- providing a header or a component of a header, and
- automatically aligning the header or the component with the housing using a reference marker to electrically contact the header or the component with said at least one feedthrough conductor, wherein the reference marker is arranged on one of: the header, said component, the housing, a tool.

Preferably, the header or the component thereof is automatically aligned with respect to the housing by a machine which is configured to optically and/or mechanically detect said reference marker. Particularly, the coordinates of a reference edge of the housing with respect to the marker are stored in the machine so that the alignment may be made automatically.

According to a preferred embodiment of the method, said component is a header core having at least one receptacle for receiving a connector of an electrode lead and at least one electrical conductor for connecting an electrical contact of the header core to said at least one feedthrough condoctor of the feedthrough. Alternatively or in addition, said tool is a mandrel that is inserted into said receptacle to prevent ingress of potting or molding compound into the receptacle upon embedding the header with a potting or molding compound, particularly after said alignment.

Particularly, according to a preferred embodiment of the method, prior to providing the header or component thereof and/or the housing, arranging the reference marker on the header, component or housing is conducted by one of: potting (e.g., with an epoxy resin), injection molding, gluing, plugging, welding (e.g. ultrasonic welding), machining, spraying, printing.

Particularly, according to a preferred embodiment of the method, the reference marker is formed (e.g. by injection molding) directly on the header, particularly via a master or injection molding tool, such that the reference marker comprises a 3D surface and therefore forms a mechanical and/or optical reference marker, in particular.

In the following, embodiments of the present invention as well as further features and advantages of the present invention shall be described with references to the Figures, wherein
- Fig. 1: shows an embodiment of an implantable medical device according to the present invention, particularly different possible positions for a reference marker on a medical device in form of a cardioverter defibrillator;
- Fig. 2: shows an embodiment of an implantable medical device according to the present invention, particularly different possible positions for a reference marker on a medical device in form of a pacemaker, and
- Fig. 3: shows detail of a further embodiment, wherein the reference marker is arranged on a mandrel.

Regarding implantable medical devices 1 that comprise a header 4 that has to be connected to a housing 2 of the device 1, the upper edge (area of the header feedthrough 3) is often used as the reference edge. After header casting, this is no longer available to process development in the context of mounting external components to and in the header 4.

However, if a reference mark is placed/mounted in relation to the reference edge in/on the header 4 before header casting, a partially or fully automated device can orient itself according to this and thus achieve a significantly lower assembly deviation of the components to be mounted (e.g. polishing plugs).

At the same time, an improvement in position control during assembly of the header cores (potting cores) is possible.

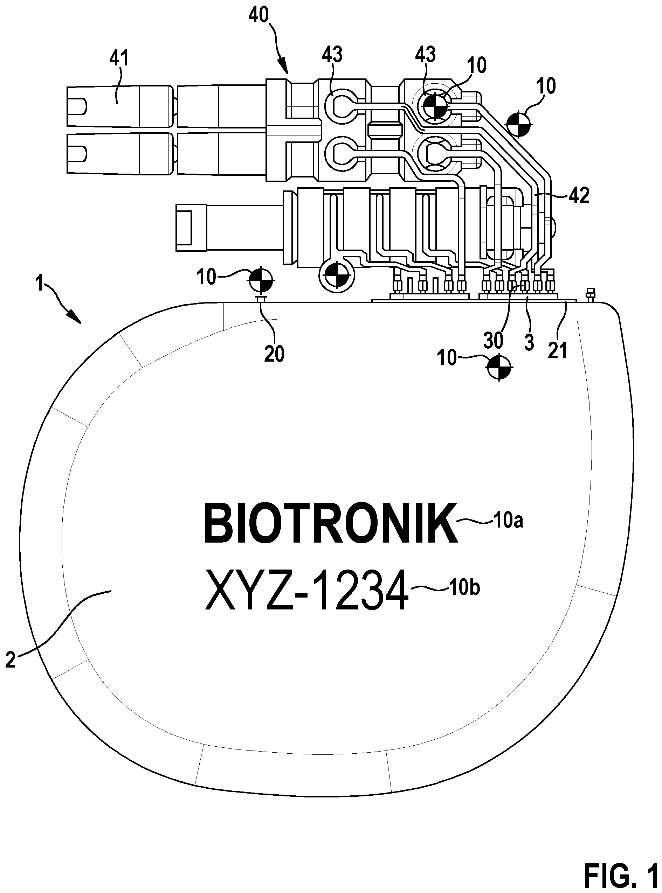

As indicated in Fig. 1 the present invention therefore suggests to provide an implantable medical device 1 (here as an example an implantable cardioverter defibrillator) that comprises a housing 2 enclosing an electronic circuit of the implantable medical device 1, the housing 2 comprising a feedthrough 3 having at least one pin 30 for electrically connecting the electronic circuit from outside the housing 2, and a header 4, the header 4 comprising a header core 40 having at least one receptacle 41 for receiving a connector of an electrode lead and at least one electrical conductor 42 for connecting an electrical contact 43 of the header core 40 to said at least one pin 30 of the feedthrough 3. Via the electrical contact 43, the receptacle 41 (e.g. a contact therein) can be electrically connected to the at least one pin (via conductor 42).

To be able to automatically align the header core 40 with respect to the housing 2 so that an electrical connection is made between the at least one pin 30 and the corresponding electrical conductor (e.g. wiring band) 42, the implantable medical device 1 comprises an optical and/or mechanical reference marker 10 arranged on the header 4, particularly on the header core 40, or on the housing 2.

Due to the reference marker 10, the header core 40 may for instance be aligned with the housing 2 to then allow to embed the properly positioned header core 40 in a potting compound 44 thus forming the final header 4. Furthermore, with the header 4 now being formed out of the potting compound 44, the reference edge indicating the position of the feedthrough 3 is now covered and thus not accessible any longer. However, due to the reference marker 10, further mounting of components on the header 4 may be achieved at positions known relative to the reference marker 10. Advantageously, the header 4, particularly the header core the housing 2 exhibit a references marker 10, respectively, in order precisely align the header 2 or header core 40 with the housing 2. Alternatively, the housing 2 comprises a manufacturers name or brand 10a and/or a product label, name or code (e.g. alphanumeric and/or barcode) preferably introduced by means of laser inscription, wherein the manufacturers name or brand 10a and/or the product label, name or code may provided a reference edge for alignment with the reference marker 10.

Particularly, Fig. 1 indicates several alternative positions for the reference marker 10, which may be arranged at an anchor 20 that will be embedded in the potting compound 44 to anchor the header 4 to the housing 2. The reference marker 10 may also be arranged on the header core 40, particularly on a contact 43 of the header core 40 or on an electrical conductor (wiring band) 41 that connects the header core 40 to the at least one feedthrough pin 30.

The reference marker 10 may also be arranged on the feedthrough 3 or on a flange 21 of the housing to which the feedthrough 3 is fixed.

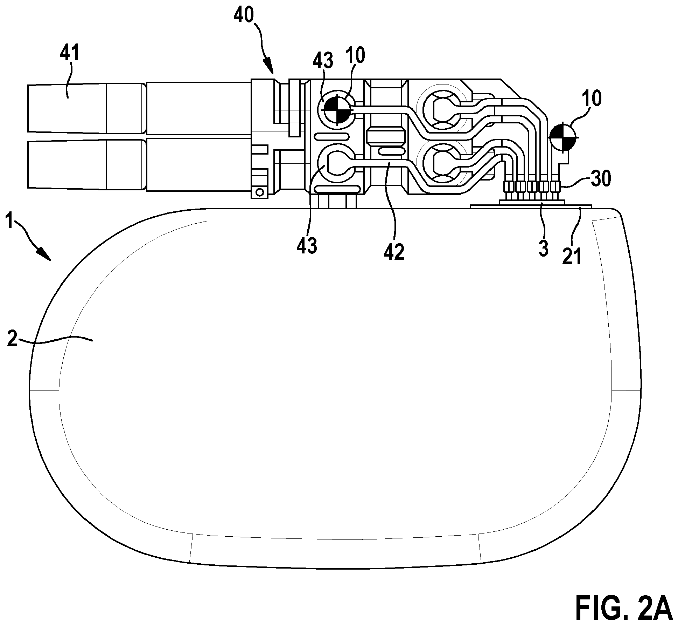

Fig. 2 (A) shows possible positions of the reference marker 10 in case of an implantable medical device 1 in form of a pacemaker. Also here, the reference marker 10 can be positioned as described in conjunction with Fig. 1.

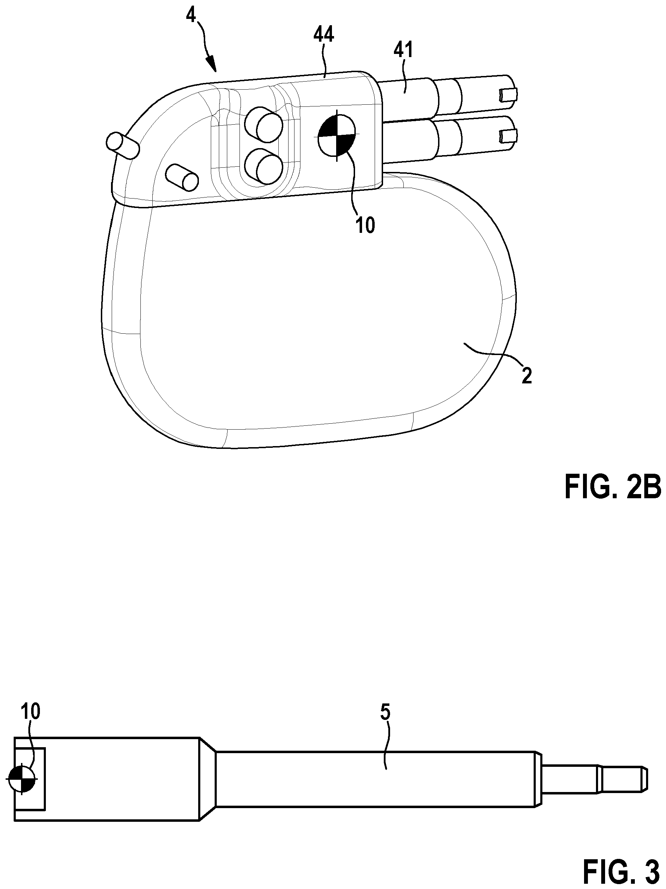

Furthermore, according to Fig. 2(B), the reference marker 10 can also be placed on the header 4, particularly as a 3D contour for a mechanical detection.

According to yet another embodiment of the present invention, as shown in Fig. 3, the reference marker 10 may also be arranged on a mandrel 5 that is used upon embedding the header core 40 in a potting compound 44 to close the receptacle 41 to prevent ingress of potting compound 44 before the latter is cured. Due to the fact that the mandrel 5 fits into the receptacle 41 with virtually no play, the reference marker 10 can also be provided on such an external tool 5.

Furthermore, according to a preferred embodiment, the reference marker 10 shown in Figs. 1, 2(A) can be a high contrast reference marker 10 and preferably defines a circle, the circle comprising two relatively dark sectors (e.g. black sectors) and two relatively bright sectors being brighter than the dark sectors, wherein the relatively dark and relatively bright sectors are arranged in an alternating fashion, wherein preferably each sector corresponds to an angle of 90°, i.e., corresponds to ¼ of the circle's area.

The advantage of the invention at hand is that significantly better positional accuracy can be achieved when mounding the header/header core and when mounting external components on and in the header as part of partial and full automation. At the same time, it enables one to create jigs and fixtures at low cost and time, and to shorten project times. In process development, less effort is required for the development of assembly parameters, and in ongoing production, less effort is required for rework and rejections in the area of quality assurance and production.

## Claims

1. An implantable medical device (1), comprising:
a housing (2) enclosing an electronic circuit of the implantable medical device (1), the housing (2) comprising a feedthrough (3) having at least one feedthrough conductor (30) for electrically connecting the electronic circuit from outside the housing (2), and a header (4), the header (4) comprising a header core (40) having at least one receptacle (41) for receiving a connector of an electrode lead and at least one electrical conductor (42) for connecting an electrical contact (43) of the header core (40) to said at least one feedthrough conductor (30) of the feedthrough (3),
**characterized in that**
the implantable medical device (1) comprises an optical and/or mechanical reference marker (10) arranged on the header (4) or on the housing (2), wherein the reference marker (10) is configured to allow automated alignment of the header core (40) with respect to the housing (2) during production of the implantable medical device (1) so that said at least one electrical conductor (42) contacts the at least one feedthrough conductor (30) of the feedthrough (3).

2. The implantable medical device according to one of the preceding claims, wherein the header core (40) is embedded in a potting or molding compound (44) which provides an outer surface of the header (4).

3. The implantable medical device according to one of the preceding claims, wherein the reference marker (10) is arranged on the header core (40).

4. The implantable medical device according to claims 2 and 3, wherein the potting or molding compound (44) is at least partially transparent so that the reference marker (10) is visible from outside the header (4) through the potting compound (44).

5. The implantable medical device according to claim 2, wherein the reference marker (10) is arranged on the outer surface of the potting or molding compound (44).

6. The implantable medical device according to one of the claims 1 to 2, wherein the reference marker (10) is arranged on said electrical conductor (42) or on a printed circuit board of the header (4).

7. The implantable medical device according to one of the claims 1 to 2, wherein the reference marker (10) is arranged on said electrical conductor or on a printed circuit board of the header.

8. The implantable medical device according to one of the claims 1 to 2, wherein the reference marker (10) is arranged on an anchor (20) of the housing (2), wherein the anchor anchors the header to the housing.

9. The implantable medical device according to one of the claims 1 to 2, wherein the reference marker (10) is arranged on the feedthrough (3) of the housing (2).

10. The implantable medical device according to one of the claims 1 to 2, wherein the reference marker (10) is arranged on a flange (21) of the housing (2), wherein the feedthrough (3) is fixed to the flange (21).

11. The implantable medical device according to one of the claims 1 to 2, wherein the reference marker (10) is arranged directly on a reference edge (10a, 10b) of the housing (2).

12. The implantable medical device according to one of the preceding claims, wherein the reference marker (10) comprises at least one of: a 3D shape, one or several graphical features, one or several lines, one of several points, one or several circles, one or several colors, an image, a feature detectable by X-ray.

13. An implantable medical device assembly, comprising:
- a housing (2) enclosing an electronic circuit, the housing (2) comprising a feedthrough (3) having at least one feedthrough conductor (30) for electrically connecting the electronic circuit from outside the housing (2), and
- a header (4), the header (4) comprising a header core (40) having at least one receptacle (41) for receiving a connector of an electrode lead and at least one electrical conductor (42) for connecting an electrical contact (43) of the header core (40) to said at least one feedthrough conductor (30) of the feedthrough (3),
**characterized in that**
the implantable medical device assembly comprises a mandrel (5) configured to be inserted into said receptacle (41) to prevent ingress of potting compound (44) into the receptacle (41) upon casting the header (4) with the potting compound (44), and wherein an optical and/or mechanical reference marker (10) is arranged on the mandrel (5), wherein the reference marker (10) is configured to allow automated alignment of the header core (40) with respect to the housing (2) so that said at least one electrical conductor (42) contacts the at least one feedthrough conductor (30) of the feedthrough (3).

14. A method for producing an implantable medical device (1), comprising the steps of:
- providing a housing (2) of the implantable medical device (1) enclosing an electronic circuit of the implantable medical device, the housing (2) comprising a feedthrough (3) having at least one feedthrough conductor (30) for electrically connecting the electronic circuit from outside the housing,
- providing a header (4) or a component (40) of a header, and
- automatically aligning the header (4) or the component (40) with the housing (2) using a reference marker (10) to electrically contact the header (4) or the component (40) with said at least one feedthrough conductor (30), wherein the reference marker (10) is arranged on one of: the header (4), said component (40), the housing (2), a tool (5).

15. The method according to claim 14, wherein said component (40) is a header core having at least one receptacle (41) for receiving a connector of an electrode lead and at least one electrical conductor (42) for connecting an electrical contact (43) of the header core (40) to said at least one feedthrough conductor (30) of the feedthrough (3), and/or wherein said tool (5) is a mandrel that is inserted into said receptacle (41) to prevent ingress of potting compound (44) into the receptacle (41) upon casting the header (4) with the potting compound (44).
